# EUROPÄISCHE PATENTSCHRIFT

(11) **EP 2 897 933 B1**
(45) Veröffentlichungstag und Bekanntmachung des Hinweises auf die Patenterteilung: **07.06.2017**
(21) Anmeldenummer: 13766261.5
(22) Anmeldetag: 18.09.2013
(51) Int. Cl.: C07C 263/10, C07C 265/14

(54) **VERFAHREN ZUR HERSTELLUNG VON DIISOCYANATEN DURCH PHOSGENIERUNG VON DIAMINSUSPENSIONEN**
METHOD OF PRODUCING DIISOCYANATES BY PHOSGENATION OF DIAMINE SUSPENSIONS
PROCÉDÉ DE FABRICATION DE DIISOCYANATES PAR PHOSGÉNATION DE SUSPENSIONS DE DIAMINE

(30) Priorität: 24.09.2012 EP 12185575
(43) Veröffentlichungstag der Anmeldung: 29.07.2015
(73) Patentinhaber: Covestro Deutschland AG, 51373 Leverkusen (DE)
(72) Erfinder: LAUE, Jörg, 59399 Olfen (DE); STEFFENS, Christian, 51067 Köln (DE); KRAUSE, Jens, 51375 Leverkusen (DE); WERSHOFEN, Stefan, 41065 Mönchengladbach (DE); KILIAN, Werner, 51375 Leverkusen (DE); SEEKAMP, Marc, 51061 Köln (DE); RUHLAND, Matthias, 51375 Leverkusen (DE)
(74) Vertreter: Levpat
(86) Internationale Anmeldenummer: PCT/EP2013/069347
(87) Internationale Veröffentlichungsnummer: WO 2014/044699

(56) Entgegenhaltungen:
- WO-A1-2009/013303
- DE-A1- 2 404 774

## Beschreibung

Gegenstand der Erfindung ist ein Verfahren zur Herstellung von organischen Diisocyanaten durch Umsetzung der korrespondierenden Diamine mit Phosgen in inerten Lösemitteln. Als Diamine werden hochschmelzende Diamine aus der Reihe von 1,5-Naphthalindiamin, Tetralindiamin, 1,4-Phenylendiamin, Duroldiamin und o-Tolidindiamin eingesetzt. Erfindungsgemäß wird eine Suspension der Diamine in inerten Lösungsmitteln hergestellt, wobei dynamische Mischaggregate, ausgewählt aus Dispergierscheiben und Rotor-Stator-Systemen, eingesetzt werden, und die erhaltene Suspension wird phosgeniert.

Diisocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Diamine mit Phosgen. Die kontinuierliche Herstellung von organischen Isocyanaten durch Reaktion von primären organischen Aminen mit Phosgen ist vielfach beschrieben und wird im großtechnischen Maßstab durchgeführt (siehe z. B. Ullmanns Encyclopedia of Industrial Chemistry, Wiley-VCH Verlag GmbH & Co KGaA, Weinheim, Online ISBN: 9783527306732, DOI: 10.1002/14356007.a14_611, S. 63 ff (2012)).

In der Regel wird die Phosgenierung zweistufig oder mehrstufig durchgeführt. In der ersten Stufe der Phosgenierung wird das Amin mit Phosgen zu Carbaminsäurechlorid und Chlorwasserstoff und in einer parallelen Reaktion zu Aminhydrochlorid, welches im Allgemeinen aufgrund seiner geringen Löslichkeit im Reaktionsgemisch als Feststoff ausfällt, umgesetzt. In der Folge wird dann das Aminhydrochlorid mit weiterem Phosgen zunächst zum Carbaminsäurechlorid umgesetzt. Die Carbaminsäurechloride werden schließlich durch Abspaltung von HCl in die entsprechenden Isocyanate überführt. Die Reaktion zwischen Amin und Phosgen ist sehr schnell, exotherm und läuft schon bei niedrigen Temperaturen ab. Parallel dazu laufen weitere, die Ausbeute schmälernde, Reaktionen, wie z. B. die Bildung von Harnstoffen aus Isocyanat und Amin, ab. Um Nebenprodukte und Feststoffbildung zu minimieren, müssen daher Amin und Phosgen, gegebenenfalls in Mischung mit organischem Lösungsmittel, schnell vermischt und die Reaktion möglichst rückvermischungsfrei geführt werden.

Zum Zwecke der Vermischung von Amin und Phosgen sind in der Literatur verschiedene Mischaggregate beschrieben. Zu den bekannten Mischaggregaten gehören vor allem Düsen, wie beispielsweise Ringschlitzdüsen (DE-A-1792660), Ringlochdüsen (DE-C1-3744001), Glattstrahlmischdüsen (EP-A1-0 065 727), Fächerstrahldüsen (DE-A1-2950216), Winkelstrahlkammerdüsen (DD-A7-300.168), Dreistromdüsen (DD-A1-132340), Gegenstrommischkammern (DE-B-1146872), Staudüsen (FR-E-69428) und Venturimischdüsen (DE-B-1175666). Auch Inlinemischer (US-A-3,321,283), Kreisel- oder Reaktionsmischpumpen (EP-A-0 291 819), Tubularreaktoren (US-A-3,226,410) oder Mikrostrukturmischer (EP-A1- 0 928 785) sind bekannt. In CA-A-832432 wird die Verwendung von Schallwellen zur Durchmischung beschrieben.

Als Lösungsmittel werden bei den in der Praxis durchgeführten Verfahren üblicherweise Chlorbenzol oder o-Dichlorbenzol verwendet. Diese Lösungsmittel haben sich bewährt, unter anderem weil sie inert sind, ein gutes Lösungsvermögen besitzen und hervorragend geeignet sind, um überschüssiges Phosgen wiederzugewinnen und von entstehendem Chlorwasserstoff zu trennen. Es ist jedoch möglich, auch andere, unter den Reaktionsbedingungen inerte Lösungsmittel zu verwenden.

Im Allgemeinen besteht keine Notwendigkeit, andere als diese Lösungsmittel zu verwenden. Schwierigkeiten treten jedoch dann auf, wenn das umzusetzende Amin in diesen Lösungsmitteln schwerlöslich ist.

Eine Möglichkeit zur Phosgenierung schwerlöslicher Amine besteht darin, die Amine in fester Form direkt oder als Suspension in einem Lösungsmittel mit einer Phosgenlösung zu vermischen und so zur Umsetzung zu bringen. Sie beruht darauf, dass durch die Umsetzung der gelösten AminMoleküle zum Isocyanat weitere Aminmoleküle in Lösung gehen und sich die Amin-Partikel nach und nach auflösen. Bei der Herstellung einer Dispersion nach den Verfahren des Standes der Technik können sich jedoch grobe, gegebenenfalls agglomerierte Partikel bilden, welche nur schwierig zu phosgenieren sind. Diese Partikel setzen sich gegebenenfalls im weiteren Verlauf nicht vollständig um, was neben Ausbeute- und Selektivitätsverlusten zu Verstopfungen und Ablagerungen führen kann.

DE-A1-196 51 041 beschreibt ein Verfahren, bei dem für Phosgenlösung und Aminlösung unterschiedliche Lösungsmittel eingesetzt werden. Nachteilig an diesem Verfahren ist ein zusätzlicher Aufwand für die Auftrennung des Lösungsmittelgemischs, bevor es erneut eingesetzt werden kann.

DE-A1-24 04 774 beschreibt ein Verfahren zur Herstellung von Isocyanaten, bei dem die Amine vorab in die entsprechenden Aminhydrochloride in Abwesenheit von Lösemitteln überführt werden. Die so gewonnenen Aminhydrochloride werden vor weiterer Phosgenierung auf eine mittlere Teilchengröße von 1 µm bis 100 µm zerkleinert. Danach werden die Aminhydrochloride in einem Verhältnis von mindestens 2 mol Phosgen pro Aminhydrochloridrest zum Isocyanat umgesetzt. Das beschriebene Verfahren hat den Nachteil, dass die Umsetzung des Aminhydrochlorids mit Phosgen sehr langsam ist. Dadurch steigt das benötigte Reaktionsvolumen an, was sich auf die Kosten für den Bau einer entsprechenden Anlage auswirkt. Zudem ist für die Herstellung der Aminhydrochloride verflüssigter Chlorwasserstoff notwendig, was einen erhöhten Aufwand für Kompression und Verflüssigung des Chlorwasserstoffs bedeutet. Ein besonderer Nachteil ist, dass mit sehr hohen Drücken von 10 bis 60 bar gearbeitet werden muss. Ein weiterer großer Nachteil ist die Unwirtschaftlichkeit des Verfahrens, da in zwei Stufen gearbeitet werden muss. In der ersten Stufe wird das Aminhydrochlorid isoliert, in der zweiten erfolgt die weitere Umsetzung zum Diisocyanat.

In DE-C-949227 wird ein Kalt-Heißphosgenierverfahren zur kontinuierlichen Herstellung von Isocyanaten durch Umsetzung von Aminen mit Phosgen in flüssiger Phase in Gegenwart eines Lösungsmittel beschrieben, bei dem in der Kaltphosgenierung eine Lösung oder Aufschlämmung des Amins in einem inerten Lösungsmittel mit flüssigen Phosgen oder einer Lösung von Phosgen in einem inerten Lösungsmittel kontinuierlich und ohne Außenkühlung in einer Mischvorrichtung unter intensivem Rühren zusammengebracht und das so erhaltene Reaktionsgemisch dann der Heißphosgenierung unterworfen wird. Als Mischeinrichtung für die Vermischung von Amin und Phosgen werden Turbomischer und Kreiselpumpe und generell Mischeinrichtungen mit mechanisch bewegten Teilen beansprucht. Die Verweilzeit in der Mischeinrichtung beträgt wenige Sekunden bis eine Minute. Beschrieben wird die Vermischung der Eduktströme. Zur Herstellung der Aminlösung bzw. Aminaufschlämmung wird keine explizite Angabe gemacht.

Die Vervollständigung der Reaktion zum Isocyanat kann in einer oder mehreren Stufen erfolgen. Beispiele für mögliche Ausführungsformen werden in DE-A1-10260082 beschrieben.

Es bestand daher ein Bedarf an einem verbesserten Verfahren zur Herstellung von hochschmelzenden organischen Diisocyanaten aus der Reihe 1,5-Naphthalindiisocyanat, 1,4-Phenylendiisocyanat, Tetralindiisocyanat, o-Tolidindiisocyanat sowie Duroldiisocyanat durch Umsetzung der entsprechenden Diamine mit Phosgen Insbesondere sollte eine Minimierung der Nebenproduktbildung und damit einhergehend Maximierung der Ausbeute sichergestellt werden.

Ein Gegenstand der vorliegenden Erfindung ist daher ein Verfahren zur Herstellung eines Diisocyanats ausgewählt aus der Gruppe bestehend aus Naphthalindiisocyanat, Phenylendiisocyanat, Tetralindiisocyanat, o-Tolidindiisocyanat und Duroldiisocyanat, wobei Naphthalindiisocyanat bevorzugt ist, umfassend die folgenden Schritte:
(i) Herstellung einer Suspension des korrespondierenden Diamins in einem inerten Lösungsmittel, wobei das Diamin mittels eines dynamischen Mischaggregats in dem Lösungsmittel verteilt wird,
(ii) Phosgenierung des in dem inerten Lösungsmittel suspendierten Diamins unter Erhalt des jeweiligen Diisocyanats,
wobei das dynamische Mischaggregat in Schritt (i) ausgewählt ist aus der Gruppe bestehend aus Dispergierscheiben und Rotor-Stator-Systemen, bevorzugt Rotor-Stator-Systemen, besonders bevorzugt Kolloidmühlen, Zahndispergiermaschinen und Drei-Walzenstühlen. Ganz besonders bevorzugt sind Zahndispergiermaschinen als dynamische Mischaggregate.

Bei den erfindungsgemäß einzusetzenden *Diaminen* ist, sofern nicht ausdrücklich anders angegeben, nicht wesentlich, welche Isomere vorliegen. Das erfindungsgemäße Verfahren ist grundsätzlich auf beliebige Isomerengemische anwendbar. Bevorzugt wird jedoch in Schritt (i) im Falle des Naphthalindiisocyanats das 1,5-Isomer (1,5-Naphthalindiisocyanat) und im Falle des Phenylendiisocyanats das 1,4-Isomer (1,4-Phenylendiisocyanat) aus dem korrespondierenden Diamin 1,5-Naphthalindiamin bzw. 1,4-Phenylendiamin hergestellt. o-Tolidindiisocyanat hat die Formel:

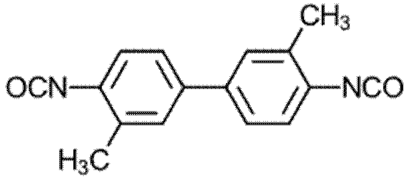

Unter *Dispergierscheiben* im Sinne der vorliegenden Erfindung sind spezielle scheibenförmige Rührorgane mit unterschiedlichen Formen und Verzahnungen zu verstehen. Hauptsächlich werden Dissolverscheiben zur Dispergierung fester Partikel in eine kontinuierliche Phase verwendet. [Siehe: "http://onlinelibrary.wiley.com/doi/10.1002/cite.200600040/pdf"] Beispiele verschiedener Dispergierscheiben sind Hochleistungs-Rührscheiben, Feinzahnscheiben, Flügelscheiben, Turbinen-Scheiben. [Siehe: http://www.berndt-scheiben.de/]

Unter *Rotor-Stator-Systemen* im Sinne der vorliegenden Erfindung sind Mischaggregate zu verstehen, die durch Kombination von rotierenden und feststehenden Elementen eine hohe Scher- und Schubbeanspruchung bewirken. Diese Technik ermöglicht das gleichmäßige Verteilen von festen (bspw. Füllstoffen) oder flüssigen Medien in einer flüssigen Matrix. Beispiele derartiger Rotor-Stator-Systeme sind Kolloidmühlen, Zahndispergiermaschinen und Drei-Walzenstühle. Drei-Walzenstühle sind beispielsweise aus der Druckfarbenherstellung als Anlagen zum Entlüften der in Rührwerkskugelmühlen dispergierten Druckfarbe bekannt. Eine solche Apparatur besteht aus drei großen Zylindern, die mit unterschiedlicher Geschwindigkeit gegeneinander laufen. Auch im pharmazeutischen Rezepturbereich werden Drei-Walzenstühle zum Desagglomerieren und Homogenisieren des Ausgangsmaterials verwendet.

Beide Typen von Mischaggregaten ermöglichen hohe Mischleistungen und das Einstellen einer definierten Partikelgrößenverteilung der suspendierten Diaminpartikel. Die genannten Mischaggregate sind ausführlich in Rotor-Stator and Disc Systems for Emulsification Processes; Kai Urban, Gerhard Wagner, David Schaffner, Danny Röglin, Joachim Ulrich; Chemical Engineering & Technology, 2006, Vol. 29, Nr. 1, Seiten 24 bis 31 sowie in DE-A1-10 2005 006 765, DE-A1-197 20 959 und US-A- 3,054,565 beschrieben.

Nachstehend wird die Erfindung detailliert erläutert. Dabei sind verschiedene Ausführungsformen beliebig miteinander kombinierbar, sofern sich für den Fachmann aus dem Kontext nicht eindeutig das Gegenteil ergibt.

In **Schritt (i)** des erfindungsgemäßen Verfahrens wird eine Suspension des Diamins in einem inerten Lösungsmittel erzeugt. Geeignete inerte Lösungsmittel sind aromatische Lösungsmittel, die auch halogeniert sein können. Beispiele hierfür sind Toluol, Monochlorbenzol, o-, m- oder p-Dichlorbenzol, Trichlorbenzol, Chlortoluole, Chlorxylole, Chlorethylbenzol, Chlornaphthaline, Chlordiphenyle, Xylole, Dekahydronaphthalin, Benzol oder Gemische der vorstehenden Lösungsmittel. Weitere Beispiele für geeignete organische Lösungsmittel sind Methylenchlorid, Perchlorethylen, Hexan, Diethylisophthalat, Tetrahydrofuran (THF), Dioxan, Trichlorfluormethan, Butylacetat und Dimethylformamid (DMF). Bevorzugt wird Monochlorbenzol oder o-Dichlorbenzol oder eine Mischung aus beiden eingesetzt; besonders bevorzugt wird Monochlorbenzol eingesetzt.

Die Erzeugung der Diaminsuspension erfolgt erfindungsgemäß unter Einsatz dynamischer Mischaggregate mit hoher Mischleistung. Diese sind ausgewählt aus Dispergierscheiben und Rotor-Stator-Systemen, bevorzugt Rotor-Stator-Systemen, besonders bevorzugt Kolloidmühlen, Zahndispergiermaschinen und Drei-Walzenstühlen. Ein Beispiel für eine Zahndispergiermaschine ist ein Ultra-Turrax-Rührer. Geeignete dynamische Mischaggregate (Rotor-Stator-Systeme) sind beispielsweise von der Firma Ystral erhältlich.

Es ist weiterhin möglich, die Bildung der Suspension in Schritt (i) durch Änderung der Löslichkeit des Diamins in dem gewählten Lösungsmittel zu erleichtern. Dies kann geschehen durch Abkühlen, Eindampfen, Fällen, Filtrieren usw.

Es ist ferner möglich, die gewünschte Suspension ein- oder mehrstufig zu erzeugen. Einstufig bedeutet, dass die gewünschte Suspension in Schritt (i) durch Anwendung eines einzigen Mischaggregats erzeugt wird; werden mehrere Mischaggregate *parallel* angeordnet, fällt eine derartige Anordnung ebenfalls unter die Definition der Einstufigkeit. Wird ein Teilstrom oder der Gesamtstrom der Suspension wieder in das einzige Mischaggregat oder in die parallel angeordneten Mischaggregate zurückgeführt (Rückführung, wiederholter Durchgang durch das einzige Mischaggregat oder die parallel angeordneten Mischaggregate), fällt diese Betriebsweise ebenfalls unter die Definition der Einstufigkeit. Mehrstufig bedeutet, dass die gewünschte Suspension in Schritt (i) durch Kombination zweier oder mehrerer *nacheinander angeordneter* Mischaggregate erzeugt wird. Die Suspension kann mit aus dem Stand der Technik an sich bekannten Verfahren nach ihrer Partikelgröße in Teilströme aufgeteilt und diese in die Dispergierstufe (Schritt (i)) zurückgeführt werden. Bevorzugt erfolgt die gewünschte Einstellung der Partikelgrößenverteilung einstufig.

Bevorzugt wird Schritt (i) des erfindungsgemäßen Verfahrens so durchgeführt, dass eine definierte Partikelgrößenverteilung des suspendierten Diamins erhalten wird. Im Folgenden wird unter der Partikelgrößenverteilung die volumengewichtete Größenverteilungsfunktion verstanden (Messung der Partikelgröße mittels Laserbeugung gemäß ISO 13320). Alle genannten Kenngrößen beziehen sich ebenfalls auf diese Verteilungsfunktion, bzw. deren näherungsweise Beschreibung und Darstellung durch eine logarithmische Normalverteilungsfunktion. Die Partikelgrößenverteilung kann grundsätzlich auch mittels anderer Methoden bestimmt werden. Dazu zählt z. B. auch gravimetrische Messtechniken, wie beispielsweise Siebanalyse, Impaktor oder Zyklonkaskadenmesstechnik. Einen Überblick über disperse Systeme und verschiedene Messverfahren gibt M. Stiess "Mechanische Verfahrenstechnik 1 ", Springer-Verlag, Berlin 1995, S. 4ff. Die für die Zwecke der vorliegenden Erfindung maßgebliche Methode ist jedoch die Laserbeugung gemäß ISO 13320.

Bevorzugt weisen mindestens 99 % der Aminpartikel (volumengewichtet) in der Suspension einen maximalen Durchmesser von 1500 µm, bevorzugt 1200 µm, besonders bevorzugt 1000 µm auf. Der mittlere (volumengewichtete) Partikeldurchmesser der Aminpartikel [D(0,50)] beträgt maximal 140 µm, bevorzugt 130 µm, besonders bevorzugt 125 µm. Die Partikelgrößenverteilung bewegt sich üblicherweise in der Bandbreite sehr breit bis sehr eng. Als Maß für die Breite der Verteilung dient die auf den Median der Partikelgrößenverteilung normierte Standardabweichung σ. Für eine sehr breite Verteilung ist σ >> 1. Für eine enge Verteilung gilt σ < 1 und für eine ideal monodisperse Verteilung der Wert σ = 0.

Durch die Reaktion des Amins in der flüssigen Phase nimmt dessen Konzentration ab. Zur Wiederherstellung des Lösungsgleichgewichts löst sich das feste Amin auf. Die Auflösungsgeschwindigkeit ist der zur Verfügung stehenden Phasengrenzfläche fest-flüssig proportional. Diese wiederum ist proportional zur Partikelgrößenverteilung. Um die Auflösungsgeschwindigkeit zu steigern, sollte daher die Partikelgröße minimiert werden. Die Reaktion kann auch an der Oberfläche des Feststoffs stattfinden. Auch hier gilt, dass die Größe der Partikel so gering wie möglich sein sollte, um die für die Reaktion an der festen Phase zur Verfügung stehende Oberfläche zu maximieren. Des Weiteren wird in diesem Fall die durch den realisierbaren Partikeldurchmesser die maximale Größe der ausfallenden Aminhydrochloridpartikel beschränkt. Deshalb gilt auch hier, dass feine Aminpartikel gegenüber sehr groben Partikeln vorzuziehen sind. Im Gegensatz zum in WO-A1-2008/006775 beschriebenen Verfahren kann ist die untere Grenze der Partikelgröße nicht kritisch, da sich das bei der Reaktion entstehende Isocyanat im Lösungsmittel löst und sich nach vollständigem Umsatz zum Isocyanat eine homogene Mischung ergibt. Insbesondere ist also ein Gegenstand der vorliegenden Erfindung ein Verfahren, bei dem die Verteilung des Diamins in dem Lösungsmittel in Schritt (i) derart durchgeführt wird, dass der volumenbezogene mittlere Partikeldurchmesser [D(0,50)] des in Schritt (i) erhaltenen suspendierten Diamins maximal 140 µm, bevorzugt maximal 130 µm, besonders bevorzugt maximal 125 µm, beträgt und maximal 1,0 Volumen-% aller suspendierten Diaminpartikel, bezogen auf das Gesamtvolumen aller Diaminpartikel einen volumenbezogenen Partikeldurchmesser von größer als 1500 µm haben. Die erreichte Partikelgrößenverteilung hängt unter anderem ab von der Auswahl des Mischaggregats, der Rührgeschwindigkeit des Mischaggregats und der Häufigkeit des Durchfahrens des Mischaggregats. Es ist nicht möglich, hier allgemeingültige Angaben zu machen, da die erreichte Partikelgrößenverteilung sehr stark von den konkreten Bedingungen abhängt. Geeignete Bedingungen können durch einfache Betriebsversuche ermittelt werden.

Die Herstellung der Diaminsuspension kann im Batch oder kontinuierlich aus Amin und Lösungsmittel, z.B. während der Zudosierung in den für die Phosgenierung (Schritt (ii)) vorgesehenen Reaktor, erfolgen. Da die Diaminsuspension üblicherweise mehr oder weniger ausgeprägt zum Sedimentieren neigt, sollte der Zeitraum zwischen Erstellung der Diaminsuspension und dem Transfer in den Phosgenierreaktor für Schritt (ii) nicht zu lang sein. Durch geeignete apparative Maßnahmen (z. B. Rühren, Umpumpen, etc.) kann ein Sedimentieren vermieden oder zumindest minimiert werden. Eine solche Maßnahme ist nicht erforderlich, wenn z. B. die Suspension durch Vermischung von Diamin und Lösungsmittel während der Zudosierung in den für die Phosgenierung vorgesehenen Reaktor in der Dosierleitung hergestellt wird.

Der Zeitraum für die Erstellung der Suspension sollte ebenfalls nicht zu lange gewählt werden, um eine (partielle) Schädigung des Amins zu vermeiden. Die Temperatur der Suspensionsbildung bzw. der resultierenden Aminsuspension beträgt bevorzugt 0 °C bis 150°C, besonders bevorzugt 0 °C bis 100 °C und ganz besonders bevorzugt 0 °C bis 70 °C. Die Erzeugung der Diaminsuspension erfolgt vorzugsweise bei einem absoluten Druck im Bereich von 1,0 bar bis 20 bar, bevorzugt von 1,0 bar bis 10 bar, besonders bevorzugt von 1,0 bis 5,0 bar erfolgen. Ein Gegenstand der vorliegenden Erfindung ist somit auch ein Verfahren, bei dem die Herstellung der Diaminsuspension in Schritt (i) bei einer Temperatur von 0 °C bis 150 °C und einem absoluten Druck von 1,0 bar bis 20 bar durchgeführt wird.

Das in Schritt (ii) benötigte Phosgen kann entweder nach Herstellung der Diaminsuspension in Schritt (i) zugegeben werden, oder, zumindest partiell, bereits in Schritt (i) vorhanden sein. Bevorzugt wird in der letztgenannten Ausführungsform das Phosgen teilweise bis vollständig in dem inerten Lösungsmittel vorgelegt, und dann erst wird das Diamin zugegeben. In dieser Ausführungsform kann auch schon während Schritt (i) in gewissem Umfang eine Phosgenierung ablaufen. Jedoch wird die Phosgenierung erst in Schritt (ii) durch Temperaturerhöhung vervollständigt.

Erfolgt die Herstellung der Suspension **vor dem Mischvorgang mit Phosgen**, muss der disperse diaminhaltige Strom dem phosgenhaltigen Strom so beigemischt werden, dass die Zeit bis zur Erreichung einer konstanten Phosgenkonzentration im Reaktionsgemisch möglichst kurz ist. Um dies zu gewährleisten, können alle dem Fachmann geläufigen technischen Methoden, wie die verteilte Zuführung des Phosgens, im Gleichstrom oder im Gegenstrom, die zentrale, axiale verdrallte Phosgeneinspeisung oder die Vermischung der Eduktströme in einer oder mehreren Düsen, wie Ringspaltdüsen oder Gegenstromdüsen, angewandt werden. Weiterhin kann es vorteilhaft sein, dynamische Mischer für die Vermischung der Diaminsuspension mit dem Phosgen einzusetzen. Mögliche Ausführungsformen werden in EP-A2-0 291 819 beschrieben (siehe insbesondere Spalte 1 Zeile 41 bis Spalte 2 Zeile 44). Vorteilhaft kann es auch sein, das Phosgen enthaltende Lösungsmittel in die Diaminsuspension einzumischen.

Eine weitere Ausführungsform der Vermischung besteht darin, das flüssige Phosgen oder eine Phosgenlösung im Reaktionsraum vorzulegen und die Diaminsuspension hinzu zu dosieren. Diese Vorgehensweise hat den Vorteil, dass der molare Überschuss an Phosgen bezogen auf die Amingruppen am Anfang sehr hoch ist und erst zum Ende der Dosierung seinen Zielwert erreicht. Dabei wird das flüssige Phosgen bzw. die Phosgenlösung bevorzugt bei Temperaturen von -40°C bis +10°C, besonders bevorzugt bei -20°C bis +0°C und ganz besonders bevorzugt von bei einer Temperatur von -10 bis 0°C vorgelegt.

Erfolgt die Herstellung der Suspension **nach dem Mischvorgang mit Phosgen**, so ist es bevorzugt, das in Schritt (ii) benötigte Phosgen zumindest teilweise, bevorzugt vollständig, bei einer Temperatur von -40°C bis +10 °C und einem absoluten Druck von 1,0 bar bis 20 bar in dem inerten Lösungsmittel vor der Zugabe des Diamins vorzulegen und die Phosgenierung durch Erhöhung der Temperatur auf einen Wert von 0 °C bis 350 °C bei einem absoluten Druck von 1,0 bar bis 20 bar nach Herstellung der Suspension zu vervollständigen.

Es ist zweckmäßig, eine effiziente Durchmischung des Reaktionsraums sicherzustellen, damit ausfallende Aminhydrochloride nicht zu schwer phosgenierbaren großen Aggregaten agglomerieren. Durch eine effiziente Vermischung kann eine hohe Raum-Zeit-Ausbeute und eine Steigerung der Qualität, insbesondere hinsichtlich der Reinheit, des NCO-Gehalts, der Molekulargewichtsverteilung und des Nebenproduktspektrums des Endprodukts realisiert werden.

Bevorzugt wird in Schritt (i) des erfindungsgemäßen Verfahrens eine Suspension des Diamins in dem inerten Lösungsmittel mit einem Gehalt von 5,0 Massen-%bis 50 Massen-%, besonders bevorzugt von 10 Massen-% bis 30 Massen-%, jeweils bezogen auf die Gesamtmasse der Diaminsuspension, hergestellt.

Eine weitere Ausführungsform kann darin bestehen, das Diamin im Gemisch mit weiteren Stoffen einzusetzen, die die Löslichkeit des Amins, der Zwischenstufen oder des Isocyanats steigern. Solche unter den Reaktionsbedingungen inerte Stoffe können z. B. polar aprotische Lösungsmittel sein, wie beispielsweise Sulfolan, Dimethylsulfoxid (DMSO) oder N-Methylpyrrolidon (NMP).

Die Phosgenierung der als Suspension vorliegenden Diamine in **Schritt (ii)** erfolgt bevorzugt bei einem absoluten Druck von 1,0 bar bis 20 bar, besonders bevorzugt von 1,0 bar bis 10 bar, ganz besonders bevorzugt von 1,0 bar bis 5,0 bar. Die Reaktionstemperatur beträgt bevorzugt 0 °C bis 350 °C, wobei zum Ende der Umsetzung die Temperatur bevorzugt 50 °C bis 250 °C, ganz besonders bevorzugt 90 °C bis 150 °C beträgt. Ein Gegenstand der vorliegenden Erfindung ist daher auch ein Verfahren, bei dem Schritt (ii) bei einer Temperatur von 0 °C bis 350 °C und einem absoluten Druck von 1,0 bar bis 5,0 bar durchgeführt wird.

Bei der Reaktion in Schritt (ii) wird Phosgen im Überschuss eingesetzt. Das heißt, pro mol Amingruppen wird mehr als ein mol Phosgen eingesetzt. Das molare Verhältnis Phosgen zu Amingruppen beträgt demnach von 1,01 : 1 bis 20 : 1, bevorzugt 1,1 : 1 bis 10 : 1, besonders bevorzugt 1,1 : 1 bis 5,0 : 1. Ggf. kann dem Reaktionsgemisch während der Umsetzung weiteres Phosgen oder Phosgenlösung zugeführt werden, um einen ausreichenden Phosgenüberschuss aufrecht zu erhalten bzw. um einen Verlust an Phosgen wieder auszugleichen.

Die Reaktion kann kontinuierlich und diskontinuierlich durchgeführt werden. Als Reaktoren kommen Rührkessel, Rohrreaktoren, Sprühtürme oder auch Schlaufenreaktoren in Betracht. Prinzipiell lassen sich aber auch andere Bauformen, die hier nicht exemplarisch aufgeführt wurden, nutzen. Bevorzugt wird diskontinuierlich gearbeitet.

Die Reaktion kann bis zum vollständigen Umsatz zum Isocyanat innerhalb der ersten Reaktionsstufe geführt werden. Es kann aber auch vorteilhaft oder notwendig sein, einen Teilumsatz, insbesondere von Resten Aminhydrochlorid, in einem Nachreaktor durchzuführen. Bei dem Nachreaktor kann es sich um übliche Reaktorbauformen unterschiedlichen Rückvermischungsgrades, wie Rührkesseln, Schlaufenreaktoren oder Rohrreaktoren handeln. Es kann weiterhin vorteilhaft sein, das Reaktionsgemisch gemäß seiner Partikelgrößenverteilung in Teilströme aufzuteilen, und getrennt einem oder mehreren Nachreaktoren zuzuführen. Als Bauformen für die Abtrennung kommen bekannte Apparate wie beispielsweise Filter, Zyklone oder Schwerkraftabscheider in Frage. Die Teilströme können dabei vor oder während der Umsetzung mit entsprechenden mechanischen Verfahren zur Einstellung der Partikelgröße behandelt werden, z. B. durch Mahlen.

Das nicht umgesetzte Phosgen wird zumeist, gegebenenfalls nach einer Reinigung, zurückgeführt und wieder zur Phosgenierung eingesetzt.

Gegenüber der herkömmlichen Flüssigphasenphosgenierung weist das hier vorgeschlagene Verfahren unter Verwendung dynamischer Mischaggregate mit hoher Mischleistung für die Herstellung der Diaminsuspension, in der bevorzugt mindestens 99 % der Aminpartikel (volumengewichtet) einen maximalen Durchmesser von 1500 µm, bevorzugt 1200 µm, besonders bevorzugt 1000 µm aufweisen, und der mittlere (volumengewichtete) Partikeldurchmesser der Aminpartikel [D(0,50)] maximal 140 µm, bevorzugt 130 µm, besonders bevorzugt 125 µm beträgt, folgende wesentliche Vorteile auf:
Die bei der Phosgenierung erhaltenen Reaktionsgemische weisen eine deutlich reduzierte Neigung zur Bildung von Nebenprodukten auf. Dadurch kann die Ausbeute des Isocyanats gesteigert werden.

Dies reduziert u.a. den Aufwand für die Aufreinigung des Isocyanats und die spezifische Abfallmenge. Sind Anlagen zur Isocyanatherstellung in der Verarbeitungskapazität durch den Abfallstrom limitiert, resultiert aus der Verringerung der Abfallmenge eine höhere erreichbare Kapazität. Durch die feine Verteilung kann der Amingehalt der Lösung bzw. der Suspension deutlich erhöht werden. Dies verringert den Aufwand für die Abtrennung und Aufarbeitung des Lösungsmittels.

Um das Diisocyanat von dem Lösemittel zu trennen bieten sich die dem Fachmann bekannten Methoden wie beispielsweise Kristallisation, Sublimation oder Destillation gegebenenfalls unter Zugabe von beispielsweise Impfkristallen oder Schleppmitteln. Bevorzugt wird ein Verfahren mit Kristallisation oder Destillation eingesetzt.

Die in Schritt (ii) erhaltenen Diisocyanate können allen dem Fachmann geläufigen Verwendungszwecken zugeführt werden. Insbesondere ist die Weiterverarbeitung mit Isocyanatreaktiven Verbindungen wie Polyolen zu Polyurethanen, ggf. über Präpolymere als Zwischenstufen, zu nennen.

Diese Polyurethane haben bevorzugt Rohdichten von 200 kg/m³ bis 1400 kg/m³, besonders bevorzugt von 600 kg/m³ bis 1400 kg/m³ und ganz besonders bevorzugt von 800 kg/m³ bis 1400 kg/m³. Ganz besonders bevorzugt werden zellige oder massive Gießelastomere hergestellt, ganz besonders bevorzugt Gießelastomere auf Polyesterpolyol Basis.

Die Isocyanatkomponente kann darüber hinaus übliche Hilfs- und Zusatzmittel, wie z.B. Rheologieverbesserer (zum Beispiel Ethylencarbonat, Propylencarbonat, dibasische Ester, Zitronensäureester), Stabilisatoren (zum Beispiel Broenstedt- und Lewis-Säuren, wie etwa Salzsäure, Phosphorsäure, Benzoylchlorid, Organomineralsäuren wie Dibutylphosphat, weiterhin Adipinsäure, Äpfelsäure, Bernsteinsäure, Traubensäure oder Zitronensäure), UV-Schutzmittel (zum Beispiel 2,6-Dibutyl-4-methylphenol), Hydrolyseschutzmittel (zum Beispiel sterisch gehinderte Carbodiimide), Emulgatoren sowie Katalysatoren (zum Beispiel Trialkylamine, Diazabicyclooctan, Zinndioctoat, Dibutylzinndilaurat, N-Alkylmorpholin, Blei-, Zink-, Zinn-, Kalzium-, Magnesiumoctoat, die entsprechenden Naphthenate und p-Nitrophenolat und/oder auch Quecksilberphenylneodecanoat) und Füllstoffe (zum Beispiel Kreide), gegebenenfalls in das/den später zu bildende/n Polyurethan/Polyharnstoff einbaufähige Farbstoffe (die also über Zerewitinoff-aktive Wasserstoffatome verfügen) und/oder Farbpigmente enthalten.

Als NCO-reaktive Verbindungen können alle dem Fachmann bekannten Verbindungen eingesetzt werden.

Als NCO-reaktive Verbindungen können Polyetherpolyole, Polyesterpolyole, Polycarbonatpolyole und Polyetheramine, welche eine mittlere OH- bzw. NH-Funktionalität von mindestens 1,5 aufweisen, sowie kurzkettige Polyole und Polyamine (Kettenverlängerer oder Vernetzer), wie sie aus dem Stand der Technik hinlänglich bekannt sind. Dies können beispielsweise niedermolekulare Diole (z.B. 1,2-Ethandiol, 1,3- bzw. 1,2-Propandiol, 1,4-Butandiol), Triole (z.B. Glycerin, Trimethylolpropan) und Tetraole (z.B. Pentaerythrit) sein, aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

Polyetherpolyole sind in an sich bekannter Weise durch Alkoxylierung von geeigneten Starter-Molekülen unter Basenkatalyse oder Einsatz von Doppelmetallcyanidverbindungen (DMC-Verbindungen) zugänglich. Geeignete Starter-Moleküle für die Herstellung von Polyetherpolyolen sind beispielsweise einfache, niedermolekulare Polyole, Wasser, organische Polyamine mit mindestens zwei N-H-Bindungen oder beliebige Gemische derartiger Starter-Moleküle. Bevorzugte Starter-Moleküle zur Herstellung von Polyetherpolyolen durch Alkoxylierung, insbesondere nach dem DMC-Verfahren, sind insbesondere einfache Polyole wie Ethylenglykol, Propylenglykol-1,3- und Butandiol-1,4, Hexandiol-1,6, Neopentylglykol, 2-Ethylhexandiol-1,3, Glyzerin, Trimethylolpropan, Pentaerythrit sowie niedermolekulare, Hydroxylgruppen aufweisende Ester derartiger Polyole mit Dicarbonsäuren der nachstehende beispielhafte genannten Art oder niedermolekulare Ethoxylierungs- oder Propoxylierungsprodukte derartiger einfacher Polyole oder beliebige Gemische derartiger modifizierter oder nicht modifizierter Alkohole. Für die Alkoxylierung geeignete Alkylenoxide sind insbesondere Ethylenoxid und/oder Propylenoxid, die in beliebiger Reihenfolge oder auch im Gemisch bei der Alkoxylierung eingesetzt werden können.

Polyesterpolyole können in bekannter Weise durch Polykondensation von niedermolekularer Polycarbonsäurederivaten, wie beispielsweise Bernsteinsäure, Adipinsäure, Korksäure, Azelainsäure, Sebacinsäure, Dodekandisäure, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, Dimerfettsäure, Trimerfettsäure, Phthalsäure, Phthalsäureanhydrid, Isophthalsäure, Terephthalsäure, Zitronensäure oder Trimellithsäure, mit niedermolekularen Polyolen, wie beispielsweise Ethylenglykol, Diethylenglykol, Neopentylglykol, Hexandiol, Butandiol, Propylenglykol, Glycerin, Trimethylolpropan 1,4-Hydroxymethylcyclohexan, 2-Methyl-1,3-propandiol, Butantriol-1,2, 4, Triethylenglykol, Tetraethylenglykol, Polyethylenglykol, Dipropylenglykol, Polypropylenglykol, Dibutylenglykol und Polybutylenglykol, oder durch Ring öffnende Polymerisation cyclischer Carbonsäureester, wie ε-Caprolacton, herstellen. Darüber hinaus lassen sich auch Hydroxycarbonsäurederivate, wie beispielsweise Milchsäure, Zimtsäure oder ω-Hydroxycapronsäure zu Polyesterpolyolen polykondensieren. Es können aber auch Polyesterpolyole oleochemischer Herkunft verwendet werden. Derartige Polyesterpolyole können beispielsweise durch vollständige Ringöffnung von epoxidierten Triglyceriden eines wenigstens teilweise olefinisch ungesättigte Fettsäure-enthaltenden Fettgemisches mit einem oder mehreren Alkoholen mit 1 bis 12 C-Atomen und anschließender partieller Umesterung der Triglycerid-Derivate zu Alkylesterpolyolen mit 1 bis 12 C-Atomen im Alkylrest hergestellt werden.

Die NCO-reaktive Verbindung kann als Vernetzerkomponente bzw. Kettenverlängerer kurzkettige Polyole bzw. Polyamine enthalten. Typische Kettenverlängerer sind Diethylentoluoldiamin (DETDA), 4,4'-Methylenbis-(2,6-diethyl)-anilin (MDEA), 4,4'-Methylenbis-(2,6-diisopropyl)-anilin (MDIPA), 4,4'-Methylen-bis-(3-chloro-2,6-diethyl)-anilin (MCDEA), Dimethylthiotoluoldiamin (DMTDA, Ethacure® 300), N,N'-Di(sec-butyl)-amino-biphenylmethan (DBMDA, Unilink^{®} 4200) oder N,N'-Di-sec-butyl-p-phenylendiamin (Unilink® 4100), 3,3'-Dichloro-4,4'-diaminodiphenylmethan (MBOCA), Trimethylenglykol-di-p-aminobenzoat (Polacure 740M). Aliphatische aminische Kettenverlängerer können ebenfalls eingesetzt oder mitverwendet werden. 1,3-Propandiol, 1,4-Butandiol, 2,3-Butandiol, 1,5-Pentandiol, 1,6-Hexandiol und HQEE (Hydrochinon-di(β-hydroxyethyl)ether) sowie Wasser. Ganz besonders bevorzugt wird 1,4-Butandiol für massive Gießelastomere und Wasser für zellige Gießelastomere verwendet.

Eine Übersicht über Polyurethane, ihre Eigenschaften und Anwendungen wird beispielsweise im Kunststoffhandbuch, Band 7, Polyurethane, 3. neu bearbeitete Auflage, Band 193, herausgegeben von Prof. Dr. G.W. Becker und Prof. Dr. D. Braun (Carl-Hanser-Verlag, München, Wien) gegeben.

Bevorzugt werden NCO-terminierte Prepolymere mit einem NCO-Gehalt von 2 bis 15 Gew.-%, ganz besonders von 2-10 Gew.-% eingesetzt. Das erfindungsgemäße Diisocyanat wird bevorzugt mit Polyolen der Funktionalität 2 bis 3 bevorzugt 2 und einer OH-Zahl von 28-112 mg KOH/g Substanz zu Prepolymeren umgesetzt. Bevorzugt werden Ester basierte Polyole eingesetzt. Die so hergestellten NCO-Prepolymere werden entweder direkt weiter umgesetzt oder als lagerstabile Prepolymere in beispielsweise Fässern bis zu ihrer endgültigen Verwendung gelagert. Bevorzugt werden 1,5-NDI basierte Prepolymere eingesetzt. Die Herstellung der Gießelastomere (Formteile) wird vorteilhaft bei einem NCO/OH Verhältnis von 0,7 bis 1,30 durchgeführt. Im Falle von zelligen Elastomeren wird die Menge des in das Formwerkzeug eingebrachten Gemisches üblicherweise so bemessen, dass die erhaltenen Formkörper die bereits dargestellte Dichte aufweisen. Die Ausgangskomponenten werden üblicherweise mit einer Temperatur von 30 bis 110°C in das Formwerkzeug eingebracht. Die Verdichtungsgrade liegen zwischen 1,1 und 8, vorzugsweise zwischen 2 und 6. Die zelligen Elastomere werden zweckmäßigerweise mit einer Niederruck-Technik oder insbesondere der Reaktionsspritztechnik (RIM) in offenen, bevorzugt geschlossenen Formwerkzeugen hergestellt.

Die Reaktionsspritzguß-Technik wird beispielsweise beschrieben von H. Piechota und H. Röhr in "Integral Schaumstoffe", Carl Hanser-Verlag, München, Wien 1975; D.J. Prepelka und J.L. Wharton in Journal of Cellular Plastics, März/April 1975, Seiten 87 bis 98 und U. Knipp in Journal of CellularPlastics, März/April 1973, Seiten 76-84.

Zusatzstoffe wie Rizinusöl oder Carbodiimide (bspw. Stabaxole der Rheinchemie als Hydrolyseschutzmittel, 2,2',6,6'-Tetraisopropyldiphenylcarbodiimid ist ein bekannter Vertreter) können sowohl dem Polyol als auch dem Prepolymer zugesetzt werden. Wasser, Emulgatoren, Katalysatoren und/oder Hilfs- und/oder Zusatzstoffe bilden mit dem Polyol gängigerweise die Polyolkomponente.

Zur besseren Entformung ist es üblich die Formwerkzeuge mit äußeren Trennmitteln zu versehen, beispielsweise Verbindungen auf Wachs- oder Silikonbasis oder wässrige Seifenlösungen. Die entformten Formkörper werden üblicherweise 1 bis 48 Stunden bei Temperaturen von 70 bis 120°C nachgetempert.

Als Emulgator werden beispielsweise sulfonierte Fettsäuren sowie weitere allgemein bekannte Emulgatoren eingesetzt, wie z. B. Polyglykolester von Fettsäuren, Alkylarylpolyglykolether, Alkoxylate von Fettsäuren, bevorzugt Polyethylenglykolester, Polypropylenglykolester, Polyethylenpolypropylenglykolester, Ethoxylate und/oder Propoxylate der Linolsäure, Linolensäure, Ölsäure, Arachidonsäure, besonders bevorzugt Ölsäureethoxylate. Alternativ können auch Polysiloxane verwendet werden. Salze von Fettsäuren mit Aminen, z.B. ölsaures Diethylamin, stearinsaures Diethanolamin, ricinolsaures Diethanolamin, Salze von Sulfonsäuren, z.B. Alkali- oder Ammoniumsalze von Dodecylbenzol- oder Dinaphthylmethandisulfonsäure sind ebenfalls bevorzugt.

Die sulfonierten Fettsäuren können bevorzugt als wässrige Lösungen, beispielsweise als 50%-ige Lösung eingesetzt werden. Typische bekannte Produkte sind Zusatzmittel SV und SM von Rheinchemie, sowie als nicht wässriger Emulgator Zusatzmittel WM von Rheinchemie.

Das Verfahren zur Herstellung der zelligen PUR-Gießelastomere wird in Gegenwart von Wasser durchgeführt. Das Wasser wirkt sowohl als Vernetzer unter Bildung von Harnstoffgruppen als auch aufgrund der Reaktion mit Isocyanatgruppen unter Bildung von Kohlendioxid als Treibmittel. Die Wassermengen, die zweckmäßigerweise verwendet werden können, betragen 0,01 bis 5 Gew.-%, vorzugsweise 0,3 bis 3,0 Gew.-%, bezogen auf das Gewicht der Komponente (b). Das Wasser kann vollständig oder teilweise in Form der wässrigen Lösungen der sulfonierten Fettsäuren eingesetzt werden.

Die Katalysatoren können einzeln wie auch in Abmischung miteinander zugegeben werden. Vorzugsweise sind dies metallorganische Verbindungen, wie Zinn-(II)-Salze von organischen Carbonsäuren, z. B. Zinn-(II)-dioctoat, Zinn-(II)-dilaurat, Dibutylzinndiacetat und Dibutylzinndilaurat und tertiäre Amine wie Tetramethyl-ethylendiamin, N-Methylmorpholin, Diethylbenzylamin, Triethylamin, Dimethylcyclohexylamin, Diazabicyclooctan, N,N'-Dimethyl-piperazin, N-Methyl-N'-(4-N-Dimethylamino-)butylpiperazin, N,N,N',N",N"-Pentamethyldiethylentriamin oder ähnliche. Weiterhin kommen als Katalysatoren in Betracht: Amidine, wie z.B. 2,3-Dimethyl-3,4,5,6-tetrahydropyrimidin, Tris-(dialkylamino-alkyl)-s-hexahydrotriazine, insbesondere Tris-(N,N-dimethylamino-propyl)-s-hexahydrotriazin, Tetraalkylammoniumhydroxide, wie z.B. Tetramethylammoniumhydroxid, Alkalihydroxide, wie z.B. Natriumhydroxid, und Alkalialkoholate, wie z.B. Natriummethylat und Kaliumisopropylat, sowie Alkalisalze von langkettigen Fettsäuren mit 10 bis 20 C-Atomen und gegebenenfalls seitenständigen OH Gruppen. Je nach einzustellender Reaktivität gelangen die Katalysatoren (b4) in Mengen von 0,001 bis 0,5 Gew.-%, bezogen auf die Komponente (a) zur Anwendung.

Verwendung finden derartige zellige PUR-Gießelastomere, auch als Formkörper bezeichnet, als Dämpfungselemente im Fahrzeugbau, beispielsweise im Automobilbau, z. B. als Zusatzfedern, Anschlagpuffer, Querlenkerlager, Hinterachsenfahrschemellager, Stabilisator-Lager, Längsstreben-Lager, Federbein-Stützlager, Stoßdämpferlager, Lager für Dreieckslenker und als auf der Felge befindliches Notrad, das beispielsweise bei einem Reifenschaden bewirkt, dass das Fahrzeug auf dem zelligen Elastomer fährt und steuerbar bleibt. Die massiven Gießelastomere können auch als Beschichtung für Rollen, Räder und Walzen, Rakel, Siebe oder Hydrozyklone verwendet werden.

### Beispiele:

### Beispiel 1 (Vergleichsbeispiel): Herstellung einer Suspension von 1,5-Naphthalindiamin (1,5-NDA) unter Verwendung eines Blattrührers

Unter Stickstoffatmosphäre wurde aus 50 g pulverförmigem, festem 1,5-NDA und 150 g trockenem Chlorbenzol (MCB) durch 10-minütiges Rühren mit einem Blattrührer bei 400 Umdrehungen/min eine Suspension hergestellt. Die resultierende Partikelgrößenverteilung ist in Tabelle 1 dokumentiert (Messung der Partikelgröße mittels Laserbeugung gemäß ISO 13320).

### Beispiel 2 (erfindungsgemäß): Herstellung einer Suspension von 1,5-NDA unter Verwendung eines Ultra-TURRAX

Unter Stickstoffatmosphäre wurde aus 50 g pulverförmigem, festem 1,5-NDA und 150 g trockenem Chlorbenzol (MCB) durch 1-minütiges Rühren mit einem Ultra-TURRAX (einem Rotor-Stator-System, nämlich einer Zahndispergiermaschine) bei 10000 Umdrehungen/min eine Suspension hergestellt. Die resultierende Partikelgrößenverteilung ist in Tabelle 1 dokumentiert (Messung der Partikelgröße mittels Laserbeugung gemäß ISO 13320).

**Tabelle 1: Partikelgrößenverteilung in den Beispielen 1 und 2**

| Verteilung | Beispiel 1: Blattrührer [µm] | Beispiel 2: Ultra-TURRAX [µm] | Reduktion der Partikelgröße in Beispiel 2 gegenüber Beispiel 1 um |
|---|---|---|---|
| D(0,06) | 40,7 | 30,9 | 24 % |
| D (0,10) | 52,8 | 40,4 | 23 % |
| D (0,15) | 64,9 | 50,4 | 22 % |
| D(0,20) | 75,9 | 59,7 | 21 % |
| D(0,30) | 97,1 | 78,1 | 20 % |
| D(0,50) | 144,9 | 121,6 | 16 % |
| D(0,85) | 321,0 | 291,9 | 9 % |
| D(0,90) | 383,5 | 352,3 | 8 % |

Somit liegt die Reduzierung der mittleren Teilchengröße bei ca. 8 bis 24 %, abhängig von der Teilchengröße.

### Beispiel 3 (Vergleichsbeispiel): Phosgenierung einer Suspension von 1,5-NDA, die unter Verwendung eines Blattrührers hergestellt wurde

In einer Phosgenierapparatur wurde bei 0 °C eine Lösung von 120 g Phosgen in 300 g Chlorbenzol hergestellt, indem Phosgen aus einer Druckgasflasche in Chlorbenzol kondensiert wurde. Unter Rühren wurde zu der Phosgenlösung in einem Guss eine gemäß Beispiel 1 hergestellte Suspension von 50 g technischem 1,5-Diaminonaphthalin (Reinheit 99,2 % laut GC) in 150 g Chlorbenzol zugegeben. Anschließend wurde unter kontinuierlichem Durchleiten von gasförmigem Phosgen aus der Druckgasflasche (ca. 5-10 l/h) das Reaktionsgemisch im Verlauf von 120 min zum Rückfluss erhitzt und 10 h bei Rückfluss gehalten. Daraufhin wurde überschüssiges Phosgen entfernt, indem im Wasserstrahlvakuum Phosgen und ein Teil des Chlorbenzols abdestilliert wurde. Danach wurde das Gemisch im Vakuum fraktioniert destilliert. Die Ausbeute an 1,5-Naphthalindiisocanat (1,5-NDI) betrug 75,6 % der Theorie.

### Beispiel 4 (erfindungsgemäß): Phosgnierung einer Suspension von 1,5-NDA, die unter Verwendung eines Ultra-TURRAX hergestellt wurde

In einer Phosgenierapparatur wurde bei 0 °C eine Lösung von 120 g Phosgen in 300 g Chlorbenzol hergestellt, indem Phosgen aus einer Druckgasflasche in Chlorbenzol kondensiert wurde. Unter Rühren wurde zu der Phosgenlösung in einem Guss eine gemäß Beispiel 2 hergestellte Suspension von 50 g technischem 1,5-Diaminonaphthalin (Reinheit 99,2 % laut GC) in 150 g Chlorbenzol zugegeben. Anschließend wurde unter kontinuierlichem Durchleiten von gasförmigem Phosgen aus der Druckgasflasche (ca. 5-10 l/h) das Reaktionsgemisch im Verlauf von 120 min zum Rückfluss erhitzt und 10 h bei Rückfluss gehalten. Daraufhin wurde überschüssiges Phosgen entfernt, indem im Wasserstrahlvakuum Phosgen und ein Teil des Chlorbenzols abdestilliert wurde. Danach wurde das Gemisch im Vakuum fraktioniert destilliert. Die Ausbeute an 1,5-NDI betrug 82,8 % der Theorie.

Ein Vergleich der Beispiele 3 und 4 verdeutlicht, dass die Verwendung eines hocheffektiven Mischaggregats zu Herstellung der 1,5-NDA-Suspension in Chlorbenzol zu einer deutlichen Verbesserung der Ausbeute an 1,5-NDI führt.

## Patentansprüche

1. Verfahren zur Herstellung eines Diisocyanats ausgewählt aus der Gruppe bestehend aus Naphthalindiisocyanat, Phenylendiisocyanat, Tetralindiisocyanat, o-Tolidindiisocyanat und Duroldiisocyanat umfassend die folgenden Schritte:
(i) Herstellung einer Suspension des korrespondierenden Diamins in einem inerten Lösungsmittel, wobei das Diamin mittels eines dynamischen Mischaggregats in dem Lösungsmittel verteilt wird,
(ii) Phosgenierung des in dem inerten Lösungsmittel suspendierten Diamins unter Erhalt des jeweiligen Diisocyanats,
**dadurch gekennzeichnet, dass**
das dynamische Mischaggregat in Schritt (i) ausgewählt ist aus der Gruppe bestehend aus Dispergierscheiben und Rotor-Stator-Systemen.

2. Verfahren nach Anspruch 1, bei dem das dynamische Mischaggregat in Schritt (i) ein Rotor-Stator-System ist.

3. Verfahren nach Anspruch 2, bei dem das Rotor-Stator-System ausgewählt ist aus der Gruppe bestehend aus Kolloidmühlen, Zahndispergiermaschinen und Drei-Walzenstühlen.

4. Verfahren nach Anspruch 3, bei dem das Rotor-Stator-System eine Zahndispergiermaschine ist.

5. Verfahren nach einem der Ansprüche 1 bis 4, bei dem die Verteilung des Diamins in dem Lösungsmittel in Schritt (i) derart durchgeführt wird, dass der volumenbezogene mittlere Partikeldurchmesser D(0,50) des in Schritt (i) erhaltenen suspendierten Diamins maximal 140 µm beträgt und maximal 1,0 Volumen-% aller suspendierten Diaminpartikel, bezogen auf das Gesamtvolumen aller Diaminpartikel einen volumenbezogenen Partikeldurchmesser von größer als 1500 µm haben,

6. Verfahren nach einem der Ansprüche 1 bis 5, bei dem Schritt (i) bei einer Temperatur von 0 °C bis 150 °C und einem absoluten Druck von 1,0 bar bis 20 bar durchgeführt wird.

7. Verfahren nach einem der Ansprüche 1 bis 6, bei dem Schritt (ii) bei einer Temperatur von 0 °C bis 350 °C und einem absoluten Druck von 1,0 bar bis 5,0 bar durchgeführt wird.

8. Verfahren nach einem der Ansprüche 1 bis 7, bei dem das in Schritt (ii) benötigte Phosgen zumindest teilweise bei einer Temperatur von -40 °C bis +10 °C und einem absoluten Druck von 1,0 bar bis 20 bar in dem inerten Lösungsmittel vor der Zugabe des Diamins vorgelegt und die Phosgenierung durch Erhöhung der Temperatur auf einen Wert von 0 °C bis 350 °C bei einem absoluten Druck von 1,0 bar bis 20 bar nach Herstellung der Suspension vervollständigt wird.

9. Verfahren nach einem der Ansprüche 1 bis 8, bei dem das Diisocyanat Naphthalindiisocyanat ist.

## Claims

1. Process for preparing a diisocyanate selected from the group consisting of naphthalene diisocyanate, phenylene diisocyanate, tetralin diisocyanate, o-tolidine diisocyanate and durene diisocyanate, which comprises the following steps:
(i)production of a suspension of the corresponding diamine in an inert solvent, with the diamine being dispersed in the solvent by means of a dynamic mixing apparatus,
(ii)phosgenation of the diamine suspended in the inert solvent to give the respective diisocyanate,
**characterized in that**
the dynamic mixing apparatus in step (i) is selected from the group consisting of dispersing discs and rotor-stator systems.

2. Process according to Claim 1, wherein the dynamic mixing apparatus in step (i) is a rotor-stator system.

3. Process according to Claim 2, wherein the rotor-stator system is selected from the group consisting of colloid mills, toothed dispersing machines and three-roll mills.

4. Process according to Claim 3, wherein the rotor-stator system is a toothed dispersing machine.

5. Process according to any of Claims 1 to 4, wherein the dispersing of the diamine in the solvent in step (i) is carried out in such a way that the volume-based average particle diameter D(0.50) of the suspended diamine obtained in step (i) is not more than 140 µm and not more than 1.0% by volume of all suspended diamine particles, based on the total volume of all diamine particles, have a volume-based particle diameter of greater than 1500 µm.

6. Process according to any of Claims 1 to 5, wherein step (i) is carried out at a temperature of from 0°C to 150°C and an absolute pressure of from 1.0 bar to 20 bar.

7. Process according to any of Claims 1 to 6, wherein step (ii) is carried out at a temperature of from 0°C to 350°C and an absolute pressure of from 1.0 bar to 5.0 bar.

8. Process according to any of Claims 1 to 7, wherein the phosgene required in step (ii) is at least partly placed initially in the inert solvent at a temperature of from -40°C to +10°C and an absolute pressure of from 1.0 bar to 20 bar before addition of the diamine and the phosgenation is completed by increasing the temperature to a value in the range from 0°C to 350°C at an absolute pressure of from 1.0 bar to 20 bar after production of the suspension.

9. Process according to any of Claims 1 to 8, wherein the diisocyanate is naphthalene diisocyanate.

## Revendications

1. Procédé pour la préparation d'un diisocyanate choisi dans le groupe constitué par le diisocyanate de naphtalène, le diisocyanate de phénylène, le diisocyanate de tétraline, le diisocyanate d'o-tolidine et le diisocyanate de durène, comprenant les étapes suivantes :
(i) préparation d'une suspension de la diamine correspondante dans un solvant inerte, la diamine étant répartie au moyen d'un appareil de mélange dynamique dans le solvant,
(ii) phosgénation de la diamine en suspension dans le solvant inerte avec obtention du diisocyanate respectif,
**caractérisé en ce que**
l'appareil de mélange dynamique dans l'étape (i) est choisi dans le groupe constitué par les disques de dispersion et les systèmes de type rotor-stator.

2. Procédé selon la revendication 1, dans lequel l'appareil de mélange dynamique dans l'étape (i) est un système de type rotor-stator.

3. Procédé selon la revendication 2, dans lequel le système de type rotor-stator est choisi dans le groupe constitué par les broyeurs colloïdaux, les appareils de dispersion dentée et les laminoirs à trois cylindres.

4. Procédé selon la revendication 3, dans lequel le système de type rotor-stator est un appareil de dispersion dentée.

5. Procédé selon l'une quelconque des revendications 1 à 4, dans lequel la répartition de la diamine dans le solvant dans l'étape (i) est réalisée de manière telle que le diamètre volumique moyen de particule D(0,50) de la diamine en suspension obtenue dans l'étape (i) est d'au maximum 140 µm et au maximum 1,0% en volume de toutes les particules de diamine en suspension, par rapport au volume total de toutes les particules de diamine, présente un diamètre volumique de particule supérieur à 1500 µm.

6. Procédé selon l'une quelconque des revendications 1 à 5, dans lequel l'étape (i) est réalisée à une température de 0°C à 150°C et à une pression absolue de 1,0 bar à 20 bars.

7. Procédé selon l'une quelconque des revendications 1 à 6, dans lequel l'étape (ii) est réalisée à une température de 0°C à 350°C et à une pression absolue de 1,0 bar à 5,0 bars.

8. Procédé selon l'une quelconque des revendications 1 à 7, dans lequel le phosgène nécessaire dans l'étape (ii) est disposé au préalable au moins partiellement à une température de -40°C à +10°C et à une pression absolue de 1,0 bar à 20 bars dans le solvant inerte avant l'addition de la diamine et la phosgénation est complétée par augmentation de la température à une valeur de 0°C à 350°C à une pression absolue de 1,0 bar à 20 bars après préparation de la suspension.

9. Procédé selon l'une quelconque des revendications 1 à 8, dans lequel le diisocyanate est le diisocyanate de naphtalène.
